Europäisches Patentamt

European Patent Office

Office européen des brevets

(19)

(11) Publication number: **0 206 331**
A2

## (12) EUROPEAN PATENT APPLICATION

(21) Application number: 86108671.8

(22) Date of filing: 25.06.86

(51) Int. Cl.⁴: **A 61 B 17/22**

(30) Priority: 26.06.85 JP 137997/85

(43) Date of publication of application: 30.12.86
Bulletin 86/52

(84) Designated Contracting States: **AT BE CH DE FR GB IT LI LU NL SE**

(71) Applicant: **YACHIYODA SANGYO CO., LTD., 2-12-15, Shinbashi Minato-ku, Tokyo (JP)**
Applicant: **YACHIYODA KOGYO CO., LTD., No. 671, Takayanagi Shounan-machi, Higashikatsushika-gun Chiba-ken (JP)**

(72) Inventor: **Takayama, Kazuyoshi, No. 4-179, Aza Obatayama Moniwa, Sendai-shi Miyagi-ken (JP)**
Inventor: **Kuwahara, Masaaki, No. 9-11, 6-chome, Chomeigaoka, Izumi-shi Miyagi-ken (JP)**
Inventor: **Kimura, Shuzo, No. 13-10, 2-chome, Suwada, Ichikawa-shi Chiba-ken (JP)**

(74) Representative: **Schmidt-Evers, Jürgen, Dipl.-Ing. et al, Patentanwälte Dipl.-Ing. H. Mitscherlich Dipl.-Ing. K. Gunschmann Dipl.-Ing. Dr.rer.nat. W. Körber Dipl.-Ing. J. Schmidt-Evers Dipl.-Ing. W. Melzer Postfach 26 01 32, D-8000 München 26 (DE)**

(54) Apparatus for disintegrating calculus by underwater shock wave from outside human body.

(57) There is disclosed an apparatus for disintegrating a calculus (10) by underwater shock wave from outside a human body (8) of the type that there are provided a shock wave generation chamber (1) of which an inner surface is formed into a shape of a part of a pseudo-ellipsoid of revolution and which is filled with liquid (4), and a shock wave generation source (2) for generating shock wave at a first focus ($f_1$) of the pseudo-ellipsoid of revolution so that a calculus (10) in a human body (8) set at a second focus ($f_2$) of the pseudo-ellipsoid of revolution may be disintegrated by the generated shock wave. For an easy handling there is provided a flexible container (3) containing the liquid (4), and the shock wave generation chamber (1) is so positioned that the interior of the chamber (1) may be filled, through an open portion thereof, with the liquid (4) in the foregoing container (3).

ACTORUM AG

June 25, 1986

APPARATUS FOR DISINTEGRATING CALCULUS BY UNDERWATER SHOCK
WAVE FROM OUTSIDE HUMAN BODY

This invention relates to an apparatus for disintegrating a calculus existing in a human body.

As for an apparatus of this kind, there has been hitherto known such a type one that there are provided a shock wave generation chamber of which an inner surface is formed into a shape of a pseudo-ellipsoid of revolution and a human body dipped in a liquid tank which is brought into communication with an open portion of the shock wave generation chamber and is provided therein with a second focus of the pseudo-ellipsoid of revolution so that shock wave may be generated, by a spark discharge, at a first focus position of the pseudo-ellipsoid of revolution, and thereby a calculus in a human body set at the second focus position in the liquid tank may be broken by the shock wave.

The foregoing conventional apparatus is advantageous in that a patient is free from any pain mentally and physically, and a long time hospital treatment is not required for an inpatient, but the same is inconvenient in that, since a liquid tank for dipping a human body is required, it cannot be avoided that the apparatus becomes large in size and cannot be transported easily. Additionally, the same is inconvenient in that, if a patient has a skin eczema, an external injury or the like, the patient cannot be dipped in the liquid tank for treatment until recovery thereof.

A purpose of this invention is to provide a calculus disintegration apparatus free from the foregoing inconveniences.

According to this invention, in an apparatus of the type that there are provided a shock wave generation chamber of which an inner surface is formed into a shape of a part of a pseudo-ellipsoid of revolution and which ist filled with liquid, and a shock wave generation source for generating shock wave at a first focus of the pseudo-ellipsoid of revolution so that a calculus in a human body set at a second focus of the pseudo-ellipsoid of revolution may be disintegrated by the generated shock wave, it is characterized in that there is provided a flexible container containing the liquid, and the shock wave generation chamber is so positioned that the interior of the chamber may be filled, through an open portion thereof, with the liquid in the foregoing container.

Next, an operation of this invention apparatus will be explained as follows:

The flexible container containing the liquid is brought into contact with the human body, and at least either one of the human body and the shock wave generation chamber is so moved, while the flexible container is being deformed, that the calculus existing in the human body may be set at the second focus of the pseudo-ellipsoid of revolution. If, after this position setting, shock wave is generated at the first focus position of the pseudo-ellipsoid of revolution in the shock wave generation chamber, this shock wave is transmitted to the human body through the container, and a high pressure is generated at the second focus position, that is, the calculus position. Thus, the calculus is disintegrated thereby.

Embodying examples of this invention will be explained with reference to the accompanying drawings:

Fig. 1 is a sectional view of one embodying example of this invention showing a step of operations condition thereof,

Fig. 2(a) is a sectional view of an important portion of a trigger means thereof,

Fig. 2(b) is a sectional view of an important portion of a modified trigger means,

Fig. 2(c) is a sectional view of an important portion of another modified one, and

Fig. 3 - 5 are sectional views of important portions of other embodying examples of this invention.

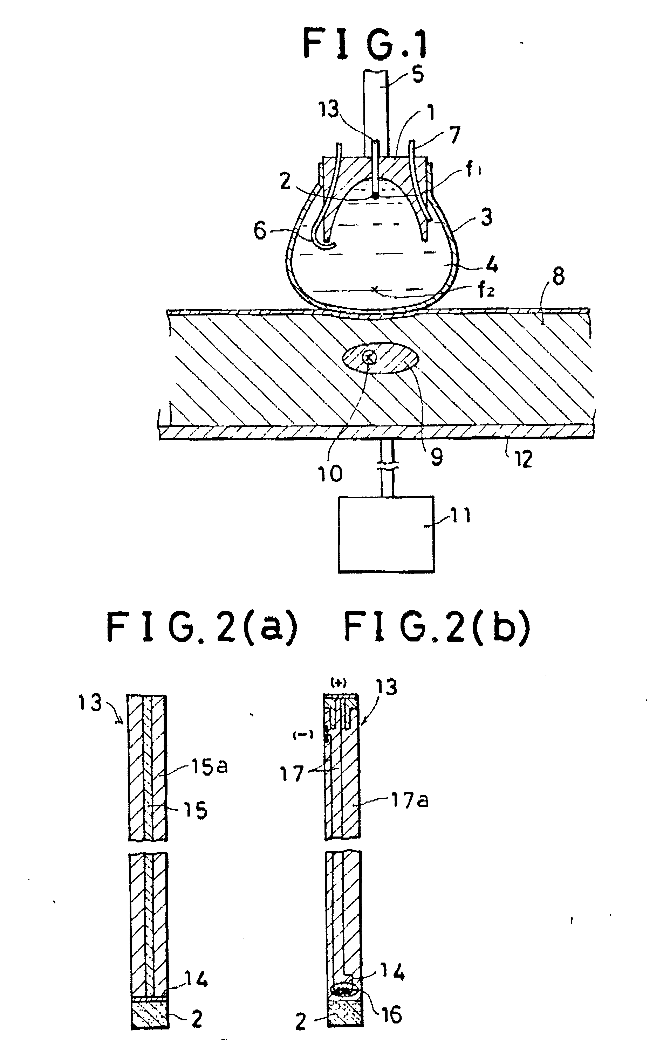

Referring to Fig. 1 showing one example thereof, numeral 1 denotes a shock wave generation chamber of which an inner surface is formed into the shape of a part of a pseudo-ellipsoid of revolution, and such a microexplosive 2 serving as a shock wave generation source that is arranged to be exploded by a trigger means 13 as described hereinafter is set at a first focus position $f_1$ of the pseudo-ellipsoid of revolution in the shock wave generation chamber 1 by being inserted into the shock wave generating chamber 1 through an opening made therein. Numeral 3 denotes a flexible container which is made of a film of synthetic rubber, synthetic resin or the like and contains liquid 4 such as water. An open mouth portion of the container 3 is mounted on the periphery of the shock wave generation chamber 1, as illustrated, and is fastened to the periphery of the shock wave generation chamber 1 by a fastening means such as a fastening band, pins or the like (not shown) applied to the periphery thereof.

The shock wave generation chamber 1 is supported on a supporting rod 5 attached to a stationary base (not illustrated), and the interior thereof is filled with the liquid 4 through an open portion thereof.

Referring to Fig. 1, numeral 6 denotes a water flow jet for removing bubbles which is so directed at its one end toward the shock wave generation chamber 1 that a jetted water may flow along on an inner surface of the shock wave generation chamber 1 for removing or sweeping bubbles which are adhered to the inner surface thereof, and is connected at its other end to a liquid tank (not illustrated), and numeral 7 denotes a pipe which is used when the liquid 4 is charged into the container 3 or is discharged for being replaced with fresh one.

Numeral 8 denotes a human body having a calculus 10 at any of the internal organs such as the kidney, the gall or the like, and the human body 8 is laid on a bed 12 which is movable in any desired direction of the horizontal X-axial and Y-axial directions and in the vertical direction by a control box 11, in order to carry out a calculus disintegrating treatment.

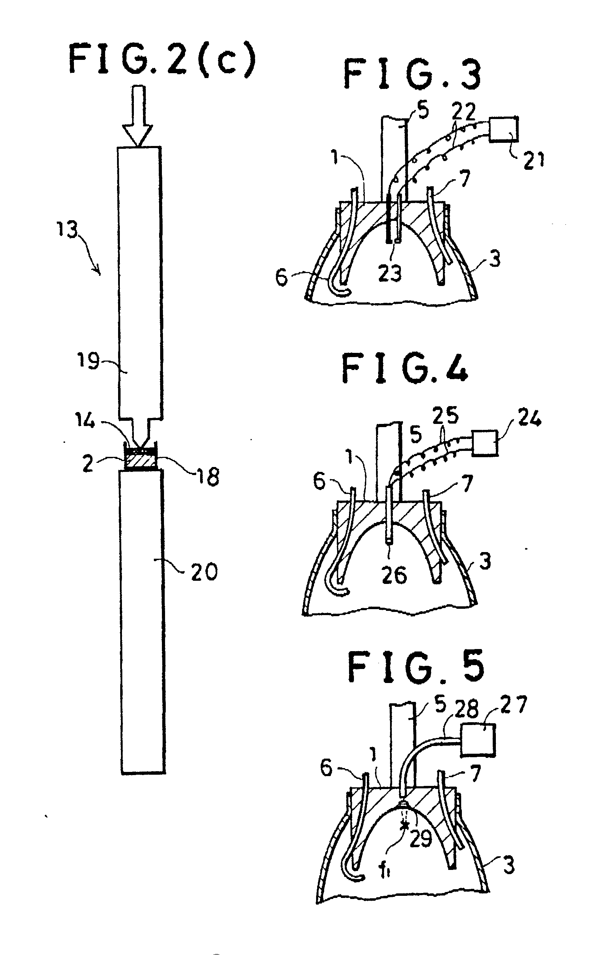

The microexplosive 2 serving as the shock wave generation source is arranged to be exploded by a trigger means 13, of which an important portion is shown, for instance, in Fig. 2(a), Fig. 2(b) or Fig. 2(c), respectively.

As for the trigger means 13, there may be used, for instance, such a type one that the same comprises, as shown in Fig. 2(a), an initiating explosive 14 which is in contact with the microexplosive 2, an optical fiber means 15 which passed through a covering tube 15a and is at its one end in contact with the initiating explosive 14, and a laser generation means (not illustrated) such as a ruby laser with a Q switch, a YAG laser with a Q switch or the like connected to the other end thereof, or such a type one that the same comprises, as shown in Fig. 2(b), an initiating explosive 14 which is laid in contact with the microexplosive 2, an electric heater 16 embedded in the initiating explosive 14, an electric wire 17 which is connected at its one end to the heater 16 and is covered, together with the initiating explosive 14, with an insulation material 17a, and a switch box (not illustrated) having a dry battery electric source of 6 - 9 V, for instance, connected to the other end thereof or such a type one that the same comprises, as shown in Fig. 2(c), an initiating explosive 14 adhered to the microexplosive 2 contained in an easily breakable casing 18, a trigger rod 19 which is placed, at its tappered forward end, on the initiating explosive 14, a receiving rod 20 for receiving the casing 18 and holding the casing 18 between the same and the trigger rod 19, and a striking means (not illustrated) for giving a blow at a high speed to the other end of the trigger rod 19 so that the forward end thereof may be introduced into the initiating explosive 14 for triggering a microexplosion of the same by mutual friction of crystals of the initiating explosive 14.

When the trigger means 13 shown in Fig. 2(c) is used, the trigger rod 19 and the receiving rod 20 are inserted and disposed horizontally in the shock wave generation chamber 1 under the condition that the microexplosive 2 and the initiating explosive 14 are held between those rods 19 and 20.

As for the microexplosive 2, there may be used such an explosive as lead azide, silver azide, tricinate, diazo dinitro phenol (D.D.N.P.) or the like.

The energy generated depending on the kind of the explosive 2 is varied, as follows:

1. lead azide     1.5 Joule/mg Specific gravity 2 g/cm$^3$
2. tricinate     1.8 Joule/mg Specific gravity 2 g/cm$^3$
3. D.D.N.P.     3.3 Joule/mg Specific gravity 1.58g/cm$^3$
4. Silver azide     1.5 Joule/mg Specific gravity 1.8g/cm$^3$

The pressure generated at the focus region when the explosive 2 is changed in weight is as described below, and is in proportion to the cube root of the weight of the explosive.

lead azide     10 mg about 3000 atmospheric pressure
lead azide     5 mg about 2500 atmospheric pressure

Accordingly, by varying the kind and the amount of the explosive, the optimum energy amount, that is the pressure value can be predetermined.

Thus, in the foregoing embodying example, the microexplosive 2 of a proper amount is set at the first focus position of the shock wave generation chamber 1, and the human body 8 laid on the bed 12 is brought to be in contact, through grease or the like, with a bottom portion of the flexible container 3.

Thereafter, under this contact condition, by operating the control box 11, the bed 12 is moved in X axial and Y axial horizontal directions and in the upper and lower directions and is so set in position that the calculus 10 of the human body 8 may be coincided with the second focus position $f_2$.

By the inventor's experiments, it has been confirmed that when the microexplosive 2 comprising lead azide of 5 mg is exploded, a calculus comprising a stone of calcium oxalate as large as 5 mm in diameter can be disintegrated by one time explosion.

In the foregoing example, the bed 12 is moved, but such a modification can be considered that the bed 12 is fixed and the shock wave generation chamber 1 is movable vertically and horizontally through a movable table (not shown) attached to the supporting rod 5 supporting the chamber 1.

In the foregoing embodying example, the microexplosive 2 is used for the shock wave generation source. However, it may be so modified that, as shown in Fig. 3, a spark gap 23 connected through an electric wire 22 to a high voltage electric source 21 is used, that, as shown in Fig. 4, a supersonic generation element 26 connected through an electric wire 25 to an electric source 24 is used, or that, as shown in Fig. 5, there is used an assembly comprising a laser means 27 such as a YAG laser with a Q switch or the like, an optical fiber means 28 for transmitting a laser beam outputted from the laser means 27, and a focusing lens 29 for focusing the laser beam outputted from the optical fiber means 28 on the first focus position $f_1$ of the pseudo-ellipsoid of revolution in the shock wave generation chamber 1.

Thus, according to this invention, it is not necessary to dip a human body into a liquid tank, so that even a patient having a disease which cannot be dipped therein can be treated, and because a liquid tank is not necessary, the apparatus can become small-sized and easy in transportation.

CLAIMS

1. An apparatus for disintegrating a calculus (10) by underwater shock wave from outside a human body (8) of the type that there are provided a shock wave generation chamber (1) of which an inner surface is formed into a shape of a part of a  pseudo-ellipsoid of revolution and which is filled with liquid (4), and a shock wave generation source (2) for generating shock wave at a first focus $(f_1)$ of the pseudo-ellipsoid of revolution so that a calculus (10) in a human body (8) set at a second focus $(f_2)$ of the pseudo-ellipsoid of revolution may be disintegrated by the generated shock wave, characterized in that there is provided a flexible container (3) containing the liquid (4), and the shock wave generation chamber (1) is so positioned that the interior of the chamber (1) may be filled, through an open portion thereof, with the liquid (4) in the foregoing container (3).

2. An apparatus as claimed in claim 1 , characterized in that at least either one of the human body (8) placed on a bed (12) and the shock wave generation chamber (1) is arranged to be movable upwards and downwards so that the flexible container (3) may be brought into contact with the human body (8) and, under this contact condition, the calculus (10) existing in the human body (8) may be set at the second focus $(f_2)$ position of the pseudo-ellipsoid of revolution.

3. An apparatus as claimed in claim 1 or 2, characterized in that the shock wave generation source comprises a microexplosive (2).

4. An apparatus as claimed in claim 1 or 2, characterized in that the shock wave generation source comprises a spark gap (23) connected to a high voltage electric source (21).

5. An apparatus as claimed in claim 1 or 2, characterized in that the shock wave generation source comprises a supersonic wave generation element (26) connected to an electric source (24).

6. An apparatus as claimed in claim 1 or 2, characterized in that the shock wave generation source comprises a laser means (27), an optical fiber means (28) for transmitting a laser beam outputted from the laser means (27), and a focusing lens (29) for focusing the laser beam outputted from the optical fiber means (28) on the first focus position ($f_1$) of the pseudo-ellipsoid of revolution in the shock wave generation chamber (1).

7. An apparatus as claimed in claim 3, characterized in that a trigger means (13) for the microexplosive (2) comprises an initiating explosive (14) which is in contact with the microexplosive (2), an optical fiber means (15) which is in contact, at its one end, with the initiating explosive (14), and a laser generation means connected to the other end of the optical fiber means.

8. An apparatus as claimed in claim 3, characterized in that a trigger means (13) for the microexplosive (2) comprises an initiating explosive (14) which is in contact with the microexplosive (2), an electric heater (16) embedded therein, an electric wire (17) which is connected,

at its one end, to the electric heater (16), and a switch box having a dry battery electric source connected to the other end thereof.

9. An apparatus as claimed in claim 3, characterized in that a trigger means (13) for the microexplosive (2) comprises an initating explosive (14) adhered to the microexplosive (2) contained in an easily breakable casing (18), a triggering rod (19) which is applied, at its tapered forward end, to the initiating explosive (14), a receiving rod (20) for holding the casing (18) between the same and the triggering rod (19), and a striking means for giving a blow at a high speed to an outer end of the triggering rod (19).

# F I G.1

# F I G.2(a)   F I G.2(b)

## FIG.2(c)

13
19
14
2
18
20

## FIG.3

5  22
1
21
7
6
23
3

## FIG.4

25  24
5
1
6
7
26
3

## FIG.5

5 28  27
1
6
7
29
$f_1$
3